# EUROPÄISCHE PATENTSCHRIFT

(11) **EP 0 698 217 B1**
(45) Veröffentlichungstag und Bekanntmachung des Hinweises auf die Patenterteilung: **06.08.1997**
(21) Anmeldenummer: 94915464.5
(22) Anmeldetag: 13.05.1994
(51) Int. Cl.: G01N 33/58, G01N 21/76, C12Q 1/28, C12Q 1/32

(54) **VERFAHREN ZUM NACHWEIS VON SUBSTANZEN**
METHOD FOR DETECTION OF SUBSTANCES
PROCEDE PERMETTANT D'IDENTIFIER DES SUBSTANCES

(30) Priorität: 12.05.1993 AT 936/93
(43) Veröffentlichungstag der Anmeldung: 28.02.1996
(73) Patentinhaber: SCHLEDERER, Thomas, A-1220 Wien (AT); FRITZ, Peter Gerald, A-5020 Salzburg (AT)
(72) Erfinder: SCHLEDERER, Thomas, A-1220 Wien (AT); FRITZ, Peter Gerald, A-5020 Salzburg (AT)
(74) Vertreter: Pawloy, Peter Michael
(86) Internationale Anmeldenummer: AT9400066
(87) Internationale Veröffentlichungsnummer: WO9427154

(56) Entgegenhaltungen:
- EP-A- 0 408 463
- WO-A-91/08490
- DE-A- 4 225 340
- ANALYTICAL BIOCHEMISTRY, Bd.136, Nr.2, 1. Februar 1984 Seiten 277 - 284 M.L.GRAYESKI ET AL. 'Determination of Fluorophor-Labelled Compounds Based on Peroxyoxalate Chemiluminescence'
- ANALYTICAL LETTERS, Bd.24, Nr.6, 1991 Seiten 1005 - 1015 M.KATAYAMA ET AL. 'Determination of hydrogen peroxide by flow injection analysis with aryl oxalate-sulforhodamine 101 chemiluminescence'
- JOURNAL OF IMMUNOLOGICAL METHODS, Bd.83, Nr.2, 7. November 1985 Seiten 317 - 325 S.TAKAYASU ET AL. 'Chemiluminescent Enzyme Immunoassay Using Beta-D-Galactosidase as the Label and the Bis(2,4,6-trichloropheny l)oxalate-Fluorescent Dye System'
- BIOMEDICAL CHROMATOGRAPHY, Bd.6, Nr.3, 1992 Seiten 149 - 154 K.NAKASHIMA ET AL. 'High Performance Liquid Chromatography with Chemiluminescence Detection of Methamphetamine and its Related Compounds Using 4-(N,N-Dimethylaminosulphonyl)-7-flu oro-2,1,3-benzoxadiazole'
- ANALYTICA CHIMICA ACTA, Bd.251, 21. Oktober 1991 Seiten 215 - 222 R.BADOR ET AL. 'Erythrosin as energy acceptor in a biphasic chemiluminescent system for glucose oxidase detection'
- Nachr.Chem.Tech.Lab. vol.40 No.5, 1992, S.547ff

## Beschreibung

Die vorliegende Erfindung betrifft ein Verfahren zum Nachweis von Substanzen mittels auf Chemilumineszenz beruhenden Nachweisreaktionen in vorzugsweise protischen Medien, gegebenenfalls in Anwesenheit basischer Katalysatoren, wobei durch die Nachweisreaktion ein Luminophor durch Elektronentransfer von seinem nicht-angeregten in einen angeregten Zustand angehoben wird und anschließend die durch Zurückfallen des Luminophors in seinen nicht-angeregten Zustand emittierte Strahlung gemessen wird.

Innerhalb der beiden letzten Jahrzehnte wurde eine Vielzahl von Technologien zum qualitativen und quantitativen Nachweis der verschiedensten Substanzen entwickelt. Dabei stellte sich insbebesondere in der Molekularbiologie sowie bei immunologischen Techniken das Problem, daß einerseits im Bereich niedrigster Konzentrationen gearbeitet werden muß und andererseits die Verwendung von radioaktiven Markern, wie sie beispielsweise bei verschiedenen Technologien des Nachweises von DNA und RNA, bzw. Fragmenten hievon, gängig ist, vermieden werden soll. Vielen der gängigen Methoden mangelt es auch daran, extrem niedrige Konzentrationen (Pico-, Femto- bzw. Attomol-Bereich) von Substanzen in biologischen Proben zu detektieren bzw. in weiterer Folge der Messung zugängliche Signale zu erzeugen.

In letzter Zeit wurde vorgeschlagen, Chemilumineszenzmethoden anstelle von Fluoreszenz und spektrophotometrischer Messungen bzw. anstelle der Messung radioaktiver Strahlung zu verwenden. Bei derartigen Chemilumineszenzverfahren wird ein mit einer chemischen Reaktion verbundener Energieumsatz der Messung zugänglich gemacht, wobei z.B. bei einem speziellen chemischen Prozeß entstehende energiereiche, instabile Zwischenstufen sofort wieder zerfallen und die dabei freiwerdende Energie durch sogenannte Luminophore oder Fluorogene in sicht- bzw. meßbare Strahlung umgewandelt wird.

So beschreibt beispielsweise die GB-PS 1 461 877 ein Verfahren zur Bestimmung eines Substrats, wobei ein spezifisches, die Reaktion des Substrats zur Bildung von Wasserstoffperoxyd katalysierendes Enzym sowie eine geeignete Chemilumineszenzverbindung einer zu testenden Probe einer Körperflüssigkeit zugesetzt werden.

Chemilumineszenzreaktionen können an sich auch mit biochemischen Reaktionen gekoppelt werden, indem die verwendeten Luminophore an ein durch beispielsweise ein Enzym abspaltbares Substrat gekoppelt und somit inaktiviert werden (welcher inaktive Substrat-Luminophor-Komplex allgemein als Fluorogen bezeichnet wird), wobei in diesem speziellen Fall dann die durch Enzymeinwirkung freigesetzte Menge an Luminophor ein Maß für die in der Probe vorhandene Menge an Enzym ist. Aufgrund der Enzymen eigenen katalytischen Aktivität kommt es dabei zu einer gewissen "enzymatischen Verstärkung", die sich darin äußert, daß die freigesetzte Menge an Luminophor ein der Inkubationszeit sowie der spezifischen Enzymaktivität entsprechendes Vielfaches der in der Probe vorhandenen Menge an Enzym ist. Der hier und im weiteren verwendete Ausdruck "Enzym" soll dabei alle Substanzen einschließen, die eine biologische Katalysatorwirkung aufweisen, insbesondere auch Antigene, Antikörper sowie Nukleinsäuremoleküle.

In einer Veröffentlichung von Irena Bronstein et al. ("Chemiluminescent Compounds for Diagnostic Tests", Research, Bd. 28, Jänner 1990, Seiten 36-39) wurde ein Chemilumineszenzsystem vorgeschlagen, wobei als Fluorogene modifizierte 1,2-Dioxetane verwendet werden, welche bei Hydrolyse Anionen produzieren, die anschließend eine weitere, in Lichtemission resultierende Zersetzung erfahren. Ein derartiges direktes Chemilumineszenzderivat enthält eine "schwache" Sauerstoff/Sauerstoff-Bindung, welche leicht zerstört wird und dadurch die Zersetzung des Fluorogens in zwei Produkte auslöst, wobei beide entstehenden Produkte eine starke, neu gebildete Carbonylgruppe enthalten. Eine dieser Carbonylgruppen befindet sich anfangs in einem elektronisch angeregten Zustand und setzt bei ihrer Rückkehr in den normalen Grundzustand etwa 420 kJ/Mol Energie frei, einiges hievon in Form von Licht. Auf diese Weise wird ein starkes Chemilumineszenzsignal erzeugt. Bei den verwendeten Verbindungen handelt es sich um zur Lumineszenz befähigte Stoffe, die nach Anregung Fluoreszenz- bzw. Phosphoreszenzlicht ausstrahlen können. Die dort beschriebenen und verwendeten Fluorogene benötigen jedoch verschiedene Substituenten, darunter einen aus einem Spiroadamantylkern bestehenden stabilisierenden Rest, eine Arylgruppe (aus welcher nach der Fragmentierung des Dioxetans Licht emittiert wird) und eine enzymlabile Schutzfunktion, welche durch das entsprechende und direkt oder indirekt zu messende Enzym abgespalten werden muß, bevor die Chemilumineszenzreaktion auftreten kann. Während der Chemilumineszenzreaktion wird also der Fluoreszenz zeigende Teil des Fluorogens zerstört. Pro Mol Fluorogen kann der Meßzyklus daher nur einmal durchlaufen werden.

Allgemein kann Chemilumineszenz auch für multiple Bestimmungen, wie z.B. in der GB-A 2 233 450 beschrieben, verwendet werden, wobei die zu bestimmenden Verbindungen jeweils mit Luminophoren verschiedener Emissionswellenlängen "markiert" werden.

Für die Anwendung der Chemilumineszenz auf analytische Probleme spielten bisher hauptsächlich die Substanzklassen der Luminolderivate, Acridiniumderivate, Phenantridiniumderivate, biogene Luciferin-Luciferasesysteme bzw. stabile Dioxetane als Luminophore eine Rolle. Herkömmliche Lumineszenzsysteme erfordern gewöhnlich alkalische oder andere Katalysatoren sowie die Anwesenheit anderer Verbindungen, welche die durch das System freigesetzte Energie verstärken, stabilisieren oder in meßbare Lichtemission überführen.

So beschreibt die GB-A-2 233 451 die Verwendung von wasserlöslichen Verstärkersubstanzen zur Stabilisierung von lichtemittierenden Fluorophoren in wässerigen Medien, wodurch die Intensität des emittierten Lichts verstärkt wird.

Die Optimierung derartiger Reaktionen durch Zugabe zusätzlicher Reagentien erwies sich jedoch bei den meisten Systemen als technisch schwer durchführbar, da zusätzliche Additive gleichzeitig zu unannehmbar hohen Hintergrundstrahlungen der verwendeten Reaktion führen, wodurch es zu Problemen bei der Messung des eigentlichen Signals kommt. Andererseits führt die Zugabe von zuwenig Additiven zu verringerten Signalen.

Es wurde auch von Campell et al. (Biochem. J., 216, Seiten 185-194 (1983)) vorgeschlagen, den zwischen einem mit einem Antigen gekoppelten Chemilumineszenz-Donor (Ag-L) und einem mit einem Antikörper gekoppelten Fluoreszenz-Akzeptor (Ak-F) stattfindende Energietransfer für ein Nachweisverfahren zu verwenden, wobei das mit dem Chemilumineszenz-Donor gekoppelte Antigen (Ag-L) mit dem zu messenden unmarkierten Antigen (Ag) um Bindung mit dem mit dem Fluoreszenz-Akzeptor gekoppelten Antikörper (Ak-F) konkurriert. Dabei kann entweder ein Komplex (L-AgAk-F) mit einer vom (Ag-L)-Komplex unterschiedlicher Emissionswellenlänge (aufgrund des möglichen Energietransfers) oder ein Komplex (AgAk-F) (bei dem kein Energietransfer möglich ist) gebildet werden. Anschließend erfolgt dann eine Messung der Strahlungsintensität bei zwei Wellenlängen, nämlich bei der Emissionswellenlänge des (Ag-L)-Komplexes sowie bei der Emissionswellenlänge des (L-AgAk-F)-Komplexes, wobei zur besseren Signaltrennung das jeweils andere Signal, welches selbst ebenfalls konzentrationsspezifisch ist, ausgefiltert wird. In weiterer Folge ergibt sich dann die (nachzuweisende) Menge an unmarkiertem Antigen aus der - allerdings bei geringen nachzuweisenden Mengen nur minimalen - Abnahme des Intensitätsverhältnisses der beiden Signale. Da es sich bei einem derartigen sogenannten "homogenen Immunoassay" um ein kompetitives System handelt, treten zusätzlich zu den eben angesprochenen Problemen beim Nachweis kleiner und kleinster Mengen einer Substanz auch noch erhebliche praktische Probleme hinsichtlich Messung der einzusetzenden Mengen an beispielsweise mit dem Chemilumineszenz-Donor gekoppelten Antigen (Ag-L) auf, welche Mengen den nachzuweisenden Mengen an unmarkiertem Antigen in etwa entsprechen müssen. Das verwendete Meßgerät, etwa ein Chemiluminometer, muß dabei auch bei beiden Wellenlängen mit entsprechenden Detektoren ausgerüstet sein. Die Nachweisgrenze dieses Systems liegt bei etwa 10⁻¹⁶ mol/100 µl.

Weiters ist aus der DD-A 280 824 ein Verfahren zur quantitativen Bestimmung von Oxalat der allgemeinen Formel R¹R²(COO)₂, wobei Oxalsäure bzw. die oxalathältige Probelösung mit einem geeigneten Oxydationsmittel in Monoperoxyoxalsäure überführt wird, deren Zerfall in Gegenwart eines Aktivators zu einer Photonenemission führt, welche mit einem Chemilumineszenzmeßgerät registriert werden kann.

Gemäß M.L. Grayeski und W.R. Seitz, Anal.Biochem. 136, (1984), S. 277-284; R. Bador und M.Morin, Anal.Chim.Acta 251, (1991), S. 215-222 sowie S. Takaysu et al., J.Immunol.Meth. 83, (1985), S. 317-325 wird die Peroxyoxalat-Chemilumineszenz als hochsensibles Nachweissystem in Immuntestverfahren ausgenützt. In den genannten Dokumenten wird die eigentliche Immunreaktion in wässerigem Medium, die nachweisende Chemilumineszenzreaktion aufgrund der geringen Löslichkeit der Oxalate in einem Gemisch von wässerigen Medien und organischen Lösungsmitteln durchgeführt.

In den Methoden gemäß Bador et al. (1991) sowie Takaysu et al. (1985) wird der jeweilige Luminophor durch Einwirkung eines geeigneten Enzyms, z.B. Glucoseoxidase oder Peroxidase aktiviert.

Desweiteren wurde gemäß M.L. Grayeski et al. (1984) versucht, die Sensitivität des Testsystems durch Ausfilterung der Hintergrundstrahlung zu steigern. Dies gelang jedoch offenbar nicht (siehe den ersten vollständigen Absatz auf Seite 281 und Tabelle 1).

Die vorliegende Erfindung betrifft nun ein Verfahren der eingangs angegebenen Art, wobei bei einer auf der oxidativen Zersetzung von gegebenenfalls substituierten Oxalaten oder Oxamiden beruhenden Chemilumineszenzreaktion ein redoxsensitives Luminophor verwendet wird, dessen beim Zurückfallen in den nicht-angeregten Zustand emittierte und zu messende Strahlung langwelliger als die unspezifische Hintergrundstrahlung der verwendeten Reaktion ist und wobei die unspezifische Hintergrundstrahlung der Reaktion durch eine geeignete Wellenlängenselektionseinheit im wesentlichen zur Gänze gelöscht wird. Bisher war das obgenannte Chemilumineszenzsystem vor allem als Chemilumineszenzdetektor für die HPLC von Interesse, da es auf Grund seiner geringen Löslichkeit und unzureichender Solvolysebeständigkeit in protischen Medien für immunologische Techniken kaum einsetzbar war. Bei Einsatz als HPLC-Detektor konnten mit derartigen, auf chemisch induzierter Elektronenaustauschlumineszenz (CIEEL) beruhenden Systemen bereits niedrige Konzentrationen an Luminophoren nachgewiesen werden, da die Oxidationsreaktion ohne Zusatz eines Luminophors um Größenordnungen weniger Licht abstrahlt als bei Zusatz eines Luminophors. Durch die erfindungsgemäße Kombination der Maßnahmen der Verwendung eines speziellen Luminophors in Verbindung mit einer geeigneten Wellenlängenselektionseinheit kann nun die Nachweisgrenze für Luminophorkonzentrationen um Größenordnungen verbessert werden.

Zugleich mit der oben erwähnten Maßnahme des Löschens der unspezifischen Hintergrundstrahlung der verwendeten Chemilumineszenzreaktion kann das zu messende Signal durch eine Verstärkung mittels chemischer bzw. enzymatischer Reaktion(en) über die unspezifische Hintergrundstrahlung der verwendeten Chemilumineszenzreaktion hinaus vervielfacht werden, indem der nicht-angeregte Luminophor vor Zugabe des zur Erzeugung energiereicher Zwischenstufen des Peroxyoxalatsystems nötigen, gegebenfalls substituierten Oxalatesters gezielt akkumuliert wird, wodurch es zu einer erheblichen "chemischen Verstärkung" des nachzuweisenden Signals kommt.

Erfindungsgemäß ist nun auch durch die Verwendung eines neuen Lösungsmittelsystems erstmals die Möglichkeit gegeben, die auf der oxidativen Zersetzung von gegebenenfalls substituierten Oxalaten oder Oxamiden beruhenden Chemilumineszenzreaktionen als Nachweisreaktion nach einem Bioassay zu verwenden, indem zunächst die biochemische Umsetzung in wässeriger Lösung stattfindet und anschließend die Chemilumineszenzreaktion durch Zugabe der hierzu nötigen Reagentien in einem geeigneten, an sich bekannten aprotischen Lösungsmittel oder Lösungsmittelsystem, beispielsweise Acetonitril, erfolgt. Dadurch ist es möglich, praktisch unbeschränkt hohe Wasserkonzentrationen zu verwenden, wobei insbesondere eine Wasserkonzentration von etwa 50 % bevorzugt wird.

Es ist auch günstig, wenn bei der Peroxyoxalat-Chemilumineszenzreaktion ein redoxsensitives Luminophor verwendet wird, dessen beim Zurückfallen in den nicht-angeregten Zustand emittierte und zu messende Strahlung langwelliger als etwa 500 nm ist und daß eine Wellenlängenselektionseinheit verwendet wird, welche die Hintergrundstrahlung zwischen etwa 350 und 540 nm im wesentlichen zur Gänze löscht. Bisher war die Peroxyoxalat-Chemilumineszenzreaktion, wie fast alle auf der oxidativen Zersetzung von gegebenenfalls substituierten Oxalaten oder Oxamiden beruhenden Chemilumineszenzreaktionen, auf aprotische Medien beschränkt und es bestand daher bereits aus diesem Grund in der Fachwelt ein Vorurteil gegen die Verwendung dieser Chemilumineszenzreaktion als Nachweisreaktion für Bioassays.

Bei der Peroxyoxalatreaktion handelt es sich um ein hochpotentes Chemilumineszenzsystem mit besonders hoher Quantenausbeute (bis 0,4 Einstein/mol), welches System zusätzlich zu dem oberwähnten Nachteil bisher auch auf Grund seiner starken Hintergrundstrahlung in einem Bereich von 350 bis 540 nm in Bioassays, also zum Nachweis geringster Mengen, nicht zum Einsatz kommen konnte.

Andererseits ergibt sich aber bei der Verwendung der Peroxyoxalatreaktion der Vorteil, daß der verwendete Luminophor nicht zerstört wird und daher den Anregungszyklus mehr als einmal durchlaufen kann. Eine Quantenausbeute mit einem Wert von mehr als 2 Einstein (bezogen auf den Luminophor) erscheint daher möglich. Durch die erfindungsgemäße Maßnahme, daß ein Luminophor verwendet wird, dessen beim Zurückfallen in den nicht-angeregten Zustand emittierte und zu messende Strahlung langwelliger als etwa 500 nm ist, und daß gleichzeitig die Hintergrundstrahlung bei etwa 350 bis 540 nm durch eine geeignete Wellenlängenselektionseinheit im wesentlichen zur Gänze gelöscht wird, wird die Hintergrundstrahlung an der Detektionswellenlänge erheblich reduziert bzw. gelöscht und die Nachweisgrenze für den verwendeten Luminophor enorm verbessert.

Als Luminophor können dabei theoretisch alle Verbindungen mit einem Emissionsspektrum langwelliger als etwa 500 nm verwendet werden, wobei insbesondere die Verwendung von Resorufinat, Tetraphenylporphyrin, Rhodamin 123, Chlorophyll und/oder (Z,Z)-16,17-Difluorboryl-13-ethyl-2,3,7,8,12,14-hexamethyl-1,17-dihydro-15H-tripyrrin-1-on bevorzugt wird. Als für das erfindungsgemäße Verfahren geeignete Wellenlängenselektionseinheiten können beispielsweise Kantenfilter, Interferenzfilter, Gitter oder Prismen zum Einsatz kommen.

Weiters ist es günstig, wenn im Verfahren der vorliegenden Erfindung bei der Peroxyoxalat-Chemilumineszenzreaktion ein redoxsensitives Luminophor verwendet wird, dessen beim Zurückfallen in den nicht-angeregten Zustand emittierte und zu messende Strahlung langwelliger als etwa 500 nm ist, welches Luminophor aus einem geeigneten Substrat unter direktem Einfluß einer nachzuweisenden Menge einer Substanz freigesetzt wird. Dadurch ist es möglich, das erfindungsgemäße hochempfindliche Nachweisverfahren zum Nachweis beliebiger Substanzen einzusetzen, wobei lediglich ein geeignetes Substrat ausgewählt werden muß, welches Substrat dann an den Luminophor gekoppelt wird und diesen dadurch inaktiviert. Während der Nachweisreaktion wird diese Inaktivierung des Luminophors dann unter Einfluß der nachzuweisenden Substanz aufgehoben, wodurch das Luminophor einer Aktivierung durch Elektronentransfer zugänglich wird.

In diesem Zusammenhang ist es besonders vorteilhaft, wenn ein aus einem Substrat und einem Luminophor bestehendes Fluorogen eingesetzt wird, wobei der Luminophor unter direktem Einfluß einer nachzuweisenden Menge eines Enzyms aktiviert bzw. vom Substrat abgespaltet wird. So ist beispielsweise bei einer Umsatzzahl von etwa 1000/s für das Enzym nach einer Inkubationszeit von 30 min eine Anzahl von bereits ca. 1,8 x 10⁶ Mol Luminophor pro Mol Enzym vorhanden, was zu einer erheblichen Verstärkung des Signals führt. Nach dieser Inkubationszeit wird sodann die Peroxyoxalatreaktion gestartet und das erzeugte Signal sofort gemessen. Dabei ist besonders vorteilhaft, daß während der Inkubationszeit keinerlei Messungen erfolgen müssen und dadurch insbesondere bei der Durchführung von Reihenuntersuchungen erhebliche Zeiteinsparungen möglich werden.

Das zur Aktivierung bzw. Freisetzung des Luminophors verwendete Enzym kann direkt oder indirekt an einen Antikörper, ein Antigen oder an eine Nukleinsäuresonde gebunden sein. In diesem Zusammenhang sei erwähnt, daß mit dem Ausdruck "indirekt gebunden" jede Bindung zwischen Enzym und Antikörper, Antigen oder Nukleinsäuresonde gemeint ist, bei der die jeweiligen Substanzen nicht direkt sondern über anorganische oder organische Spacer, wie sie beispielsweise aus der Affinitätschromatographie bekannt sind, verbunden sind.

Hinsichtlich der Enzyme zur Aktivierung bzw. Freisetzung des Luminophors und des zugehörigen Luminophor/Substratkomplexes sind ebenfalls alle bekannten Kombinationen von enzymspezifischem Substrat (an den Luminophor gebunden) und zugehörigem Enzymkomplex denkbar; insbesondere können als Enzyme alkalische Phosphatase, Meerrettichperoxidase und/oder β-Galactosidase eingesetzt werden.

Gemäß einer weiteren bevorzugten Ausführungsform der vorliegenden Erfindung ist vorgesehen, daß der verwendete Luminophor aus einem geeigneten Substrat unter indirektem Einfluß einer nachzuweisenden Menge einer Substanz aktiviert bzw. freigesetzt wird. Dadurch wird die Verwendung des erfindungsgemäßen Verfahrens in sogenannten kaskadenartigen Bestimmungsmethoden möglich, wie sie beispielsweise von der Blutgerinnungskaskade her bekannt sind, wobei in jedem einzelnen Schritt der verwendeten Kaskade eine weitere chemische und/oder enzymatische Verstärkung des durch direkte Umsetzung erzielbaren Signals möglich ist.

Vorzugsweise erfolgt der indirekte Einfluß einer nachzuweisenden Menge eines Antigens bzw. Antikörpers auf den mit einem Enzym zur Freisetzung des Luminophors gekoppelten Antikörper bzw. Antigen über ein oder mehrere weitere Antigen-Antikörper-Reaktionen. Es kommt dabei nicht nur zu einer Verstärkung des erzielbaren Signals, sondern es ist auch ein besonders spezifischer quantitativer Nachweis möglich. So kann beispielsweise zum Nachweis eines HIV-Antigens primär ein Anti-HIV-Antikörper aus Kaninchen und sekundär ein mit einem Enzym gekoppelter Anti-Kaninchen-Antikörper aus Ziege verwendet werden, welches Enzym in weiterer Folge dann das Luminophor aus dem verwendeten Luminophor-Substrat-Komplex freisetzt.

Es ist auch günstig, wenn der indirekte Einfluß einer nachzuweisenden Menge eines Antigens bzw. Antikörpers auf den mit einem Enzym zur Freisetzung des Luminophors gekoppelten Antikörper bzw. Antigen über ein oder mehrere zwischengeschaltete Substanzen erfolgt, welche mit ein oder mehreren spezifischen Erkennungsstellen für mindestens eine weitere Verbindung versehen sind, welche weitere Verbindung(en) dann mit dem jeweiligen Enzym gekoppelt sind. Ein derartiges System wäre beispielsweise das System bestehend aus mit dem Antikörper gekoppeltem Biotin und einem Avidin- oder Streptavidin-Enzymkomplex bzw. allgemein alle bekannten Kombinationen von Kopplungspartnern, wobei das für die Aktivierung bzw. Freisetzung des Luminophors verwendete Enzym an einen der Partner gebunden ist. Selbstverständlich kann auch ein umgekehrtes Nachweisverfahren verwendet werden, wobei eine Abnahme der emittierten Strahlung gegenüber einer Blindprobe durch räumliche Anwesenheit eines Quenchers gemessen wird.

Insbesondere wird durch das erfindungsgemäße Verfahren der Einsatz der Peroxyoxalat-Chemilumineszenzreaktion, welche ein sehr effizientes System darstellt, bei direkter Luminophormarkierung eines nachzuweisenden Substrats bzw. bei chemilumineszenzverstärkten Enzymimmunoassays ermöglicht. Die bisherigen Schwierigkeiten betreffend die Unverträglichkeit der Peroxyoxalatreaktion mit protischen Medien auf Grund unzureichender Solvolysebeständigkeit und geringer Löslichkeit, werden durch die Verwendung eines neuen Lösungsmittelsystems, bestehend vorzugsweise aus etwa 50 % Wasser, überwunden. So kann bei Verwendung der Peroxyoxalatreaktion als Nachweisreaktion in einem Enzymimmunoassays durch Auswahl eines geeigneten Luminophors, welches langwelliger als die Hintergrundstrahlung der verwendeten Reaktion, insbesondere langwelliger als etwa 500 nm, emittiert und gleichzeitiger Verwendung einer geeigneten Wellenlängenselektionseinheit, eine enorme Verbesserung der Nachweisgrenze für den verwendeten Luminophor und damit in weiterer Folge für die nachzuweisende Substanz erreicht werden.

Die folgenden Beispiele sollen die vorliegende Erfindung näher erläutern.

### Beispiel 1: Berechnung der Quantenausbeute Materialien und Verfahren

Reagenzien: (Z,Z)-16,17-Difluorboryl-13-ethyl-2,3,7,8,12,14-hexamethyl-1,17-dihydro-15H-tripyrrin-1-on (GR132) wurde von Doz. Grubmayr, Institut für Organische Chemie der Universität Linz, erhalten. Geblistertes Harnstoffperoxid wurde einem HIV-Antikörpertest von Organon Teknika entnommen. Bis(2,4-dinitrophenyl)oxalat (DNPO) war von Lambda Probes, Graz. Ethylacetat und Wasser waren von spektroskopischer Reinheit von Merck, Darmstadt. Die Lumineszenz wurde an einem Hamamatsu MTP-Reader gemessen, der mit einem Hoya FL Rot-Filter ausgestattet war.

### Versuch:

10 µl einer Lösung von 125 mg Harnstoffperoxid in 4 ml Wasser, 10 µl von GR132 in Ethylacetat und 200 µl einer 1mM-Lösung von DNPO in Ethylacetat wurden in ein Glasröhrchen pipettiert, wobei die erzeugten Photonen sofort am Hamamatsu MTP Reader 5 min integriert wurden. Als Leerwert wurden statt der 10 µl GR132-Lösung 10 µl reines Ethylacetat pipettiert. Zur Bestimmung der Sensitivität und der Quantenausbeute wurde eine Verdünnungsreihe von GR132 in Ethylacetat hergestellt.

Es wurde bis zu einer Konzentration von 2,34 x 10⁸ Molekülen pro Näpfchen verdünnt. Aufgrund von Verlust durch den Raumwinkel, die Optik und die Detektionseinheit wird im MTP-Reader eine Zählrate von 14 000 Quanten erwartet. Bei einem Signal/ Rauschverhältnis von 4 wurden nach Abzug des Leerwerts 30 000 Quanten gemessen. Dies ergab eine Quantenausbeute von mehr als 2, bezogen auf den Luminophor.

### Beispiel 2: Sensitivitätsverbesserung durch Anwendung eines Filters in der Peroxyoxalatreaktion.

### Materialien und Verfahren

### Reagenzien:

Bis(2,4,6-trichlorphenyl)oxalat (TCPO) wurde am Institut für Organische Chemie der Universität Linz synthetisiert und zweimal aus Ethylacetat umkristallisiert. Von Merck erhaltenes Imidazol wurde aus Ether umkristallisiert. Wasser war von Uvasol-Qualität und Acetonitril war von Promochem ChromAR. Geblistertes Harnstoffperoxid wurde einem HIV-Antikörpertest von Organon Teknika entnommen. Natrium-Resorufinat war von Lambda Probes, Graz. Die Lumineszenz wurde an einem Berthold Biolumat LB 9500T-Luminometer, sowie am gleichen Luminometer mit einem OG 590 nm-Filter von Schott (1 mm) zwischen Röhrchenkammer und Photomultiplier gemessen.

### Versuch:

Resorufin Na-Salz wurde in einem Imidazolpuffer (7 mg Imidazol in 10 ml H₂O) verdünnt. Zu 100 µl der jeweiligen Verdünnung wurden in einem Berthold-Röhrchen (passend zum LB 9500T) 10 µl einer Harnstoffperoxidlösung in Wasser (125 mg/10 ml) und 200 µl einer Lösung von TCPO in Acetonitril (1 mM) pipettiert, das Luminometer geschlossen und die Aufzeichnung im 10 s Integralmodus manuell gestartet. Die Extinktion wurde in einer Mikrotiterplatte auf einem Anthos htIII-Photometer bei 570 nm und einer Referenzwellenlänge von 620 nm bestimmt.

Die gemessenen Werte waren wie folgt:

| | | ohne Filter | | mit Filter | |
|---|---|---|---|---|---|
| Konzentration | Extinktion | Counts | S/N* | Counts | S/N |
| 1,40 x 10⁻⁶ | 0,093 | Overflow | | Overflow | |
| 3,50 x 10⁻⁷ | 0,021 | Overflow | | Overflow | |
| 8,74 x 10⁻⁸ | 0,004 | Overflow | | 420.786 | 253 |
| 2,19 x 10⁻⁸ | -0,001 | 478.395 | 2,15 | 103.309 | 62,1 |
| 5,46 x 10⁻⁹ | 0,000 | 301.335 | 1,36 | 26.864 | 16,1 |
| 1,37 x 10⁻⁹ | -0,003 | 284.350 | 1,28 | 7.636 | 4,59 |
| 3,42 x 10⁻¹⁰ | -0,001 | 237.460 | 1,07 | 3.016 | 1,81 |
| 8,54 x 10⁻¹¹ | -0,002 | 223.395 | 1,006 | 2.146 | 1,29 |
| 2,13 x 10⁻¹¹ | -0,002 | 222.090 | 1,0001 | 1.872 | 1,13 |
| 5,34 x 10⁻¹² | -0,001 | 220.700 | 0,993 | 1.710 | 1,03 |

| | | | | | |
|---|---|---|---|---|---|
| * Signal/Rauschverhältnis | | | | | |

Aus diesen Zahlen ist ersichtlich, daß die Verwendung eines Filters zur Unterdrückung der unspezifischen Hintergrundstrahlung eine Sensitivitätssteigerung um zwei Zehnerpotenzen bewirkt.

### Beispiel 3: Pd-Koproporphyrin (Vergleichsbeispiel) Materialien und Verfahren (Geräte wie in Beispiel 2)

### Reagenzien:

Pd-Koproporphyrin war von Lambda Probes, Graz. Die restlichen Reagenzien wurden bereits beschrieben. Das Luminometer war mit einem OG 590 nm Filter von Schott (1 mm) ausgestattet.

### Versuch:

Pd-Koproporphyrin wurde in Aceton/Imidazolpuffer (10⁻² M, pH=7) gelöst und eine Verdünnungsreihe im Imidazolpuffer angelegt. Zu 100 µl Puffer wurden 10 µl Harnstoffperoxidlösung (125 mg/10 ml Wasser) und 200 µl Acetonitril (1 mM TCPO) zugesetzt. Das Luminometer wurde sofort manuell gestartet und die Counts im 10 s Integralmodus gemessen.

| Konzentration | Counts | S/N |
|---|---|---|
| 8,40 x 10⁻⁷ | Overflow | |
| 2,10 x 10⁻⁷ | Overflow | |
| 5,25 x 10⁻⁸ | Overflow | |
| 1,31 x 10⁻⁸ | 677 | 4,12 |
| 3,28 x 10⁻⁹ | 285 | 1,73 |
| 8,20 x 10⁻¹⁰ | 191 | 1,07 |
| 2,05 x 10⁻¹⁰ | 171 | 1,04 |
| 5,13 x 10⁻¹¹ | 168 | 1,02 |
| 1,28 x 10⁻¹¹ | 160 | 0,98 |

Hieraus ist ersichtlich, daß mit diesem System wasserlösliches Pd-Koproporphyrin bis zu einer Detektionsgrenze von 8 x 10⁻¹⁰ Mol/l bestimmt werden kann, dies steht jedoch in keinem Vergleich zu GR132 (3,9 x 10⁻¹¹ Mol/l) oder Na-Resorufinat (2,13 x 10⁻¹¹ Mol/l).

### Beispiel 4: Bestimmung der β-Galactosidase Materialien und Verfahren (Geräte wie in Beispiel 2)

### Reagenzien:

Avidin β-Galactosidase war von Pierce, Resorufin-β-D-galactopyranosid von Lambda Probes, MgCl₂ x 6H₂O und NaCl von Merck, HEPES/BSA-Puffer von Codon.

Resorufin-β-D-galactopyranosid wird in Chloroform/Methanol (4/1) aufgenommen, an Kieselgel 60 mit Chloroform/Methanol/ Wasser (260/70/10) als Laufmittel von Verunreinigungen getrennt und an der Hochvakuumpumpe bei Raumtemperatur getrocknet. Das leicht rötlich gefärbte Produkt wird zweimal aus Uvasol-Wasser bei 60°C umkristallisiert, mit Uvasol-Wasser nachgewaschen und mit spektroskopisch reinem Acetonitril getrocknet.

### Versuch:

Es wurde eine Verdünnungsreihe von Avidin β-Galactosidase in HEPES/BSA (20 mM HEPES, 0,5 M NaCl, pH 7,5, mit 1,0 mg/ml BSA) angelegt. 10 µl aus dieser Verdünnungsreihe wurden in einer Mikrotiterplatte von Organon Teknika mit 100 µl Substratpuffer (1 x 10⁻⁴ M Resorufin-β-D-galactopyranosid, 0,1 M NaCl, 2 mM MgCl₂, 5 mM Imidazol) versetzt und 90 min bei 37°C inkubiert. Danach wurde die Extinktion gemessen, 100 µl in Röhrchen transferiert, 10 µl H₂O₂ (1 M Uvasol H₂O) zugesetzt, in das Luminometer transferiert, 100 µl einer Lösung von TCPO in Acetonitril zugesetzt (1 mM), manuell gestartet und im 10 s-Integralmodus aufgezeichnet. Für die Auswertung wurde ein Molekulargewicht der β-Galactosidase von 500 000 zugrundegelegt.

### Auswertung:

| Moleküle | Extinktion | Counts | S/N |
|---|---|---|---|
| 5,8 x 10⁸ | 0,467 | Overflow | |
| 1,5 x 10⁸ | 0,159 | Overflow | |
| 3,6 x 10⁷ | 0,051 | Overflow | |
| 9,1 x 10⁶ | 0,016 | Overflow | |
| 2,3 x 10⁶ | 0,006 | Overflow | |
| 568.432 | 0,004 | Overflow | |
| 142.108 | 0,000 | 480.304 | 2,61 |
| 35.527 | -0,001 | 335.438 | 1,82 |
| 8.882 | -0,001 | 283.060 | 1,54 |
| 2.220 | -0,001 | 243.697 | 1,32 |
| 555 | -0,003 | 199.791 | 1,09 |
| 139 | -0,001 | 183.964 | 1,00 |

Dieses Verfahren zeichnet sich durch einen sehr niedrigen Variationskoeffizienten aus, der durch Anwendung einer automatischen Zugabe sicherlich noch verbessert werden kann. Wie zu ersehen ist, können mit diesem System innerhalb von 2 h weniger als 1000 Moleküle in 100 µl bestimmt werden.

### Beispiel 5: Bestimmung der Meerrettich-Peroxidase (HRP) Materialien und Verfahren

### Reagenzien:

Anti-Human IgG-Peroxidase-Konjugat, Konjugat Diluent, Tetramethylbenzidin-Reagens und Peroxid-Reagens wurden einem Detect-HIV-Test Kit von Coulter entnommen. Dihydrorhodamin 123 war von Lambda. Die Lumineszenz wurde an einem Berthold Biolumat LB 9500T mit einem OG 530 nm-Filter (3 mm) zwischen Röhrchenkammer und Photomultiplier gemessen.

### Versuch:

In einer Organon Teknika Mikrotiter-Verdünnungsplatte wurde eine Verdünnungsreihe (1:10) von Anti-Human IgG Peroxidase-Konjugat in Konjugat Diluent angelegt. Für die photometrische Auswertung wurden jeweils 10 µl der Verdünnung zu 100 µl Substratreagenz (Tetramethylbenzidin-Reagens/Peroxid-Reagens = 1:10) pipettiert. Für die luminometrische Auswertung wurden 10 µl der Verdünnung zu 100 µl Substratreagenz (10 µl einer 10⁻³ M Lösung von Dihydrorhodamin 123 in Acetonitril und 90 µl Peroxid-Reagens) pipettiert. Beide Ansätze wurden 15 min inkubiert, danach der TMB-Ansatz bei 620 nm am Anthos-Photometer ausgewertet, 100 µl des luminometrischen Ansatzes mit 100 µl einer 0,1 mM Lösung von TCPO in Acetonitril in einem Berthold-Röhrchen im Luminometer versetzt, manuell gestartet und im 10 s Integralmodus ausgewertet.

### Auswertung:

| Willkürliche Konzentration | Extinktion | Counts | S/N |
|---|---|---|---|
| 10.000 | 0,320 | 837.889 | 200 |
| 1.000 | 0,038 | 23.363 | 5,57 |
| 100 | 0,039 | 5.711 | 1,36 |
| 10 | 0,041 | 4.001 | 0,96 |
| 1 | 0,043 | 4.374 | 1,04 |

Aus diesen Zahlen ist ersichtlich, daß mit Verwendung dieses Reagenzsystems eine Sensitivitätssteigerung der HRP um einen Faktor 100 erreicht werden kann.

### Beispiel 7: Bestimmung der alkalischen Phosphatase Materialien und Verfahren (Geräte wie in Beispiel 2)

### Reagenzien:

Streptavidin Alkalische Phosphatase Konjugat war von Pierce, Resorufinphosphat-Pyridiniumsalz war von Lambda. Der Carbonatpuffer (0,01 M, pH = 9,6, 1 mM MgCl₂) war von Codon, Wien. Die restlichen Reagenzien wurden bereits beschrieben. Im Luminometer war das OG 590 nm-Filter (1 mm).

### Versuch:

Es wurde eine Verdünnungsreihe von Streptavidin Alkalische Phosphatase Konjugat in HEPES/BSA (20 mM HEPES, 0,5 M NaCl, pH 7,5, mit 1,0 mg/ml BSA) angelegt. 10 µl aus dieser Verdünnungsreihe wurden in einer Mikrotiterplatte von Organon Teknika mit 100 µl Substratpuffer (1 x 10⁻⁵ M Resorufinphosphat-Pyridiniumsalz, 0,1 M NaCl, 1 mM MgCl₂, 0,01 M Carbonatpuffer, pH = 9,6) versetzt und 90 min bei 37°C inkubiert. Danach wurde die Extinktion gemessen (570/620 nm), 100 µl in ein Röhrchen transferiert, 10 µl H₂O₂ (1 M Uvasol H₂O) zugesetzt, in das Luminometer transferiert, 100 µl einer Lösung von TCPO in Acetonitril zugesetzt (1mm), manuell gestartet und im 10 s-Integralmodus aufgezeichnet. Für die Auswertung wurde ein Molekulargewicht der alkalischen Phosphatase von 75 000 zugrundegelegt.

### Auswertung:

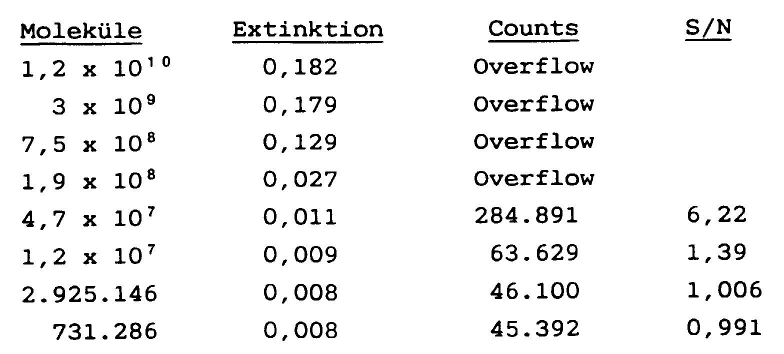

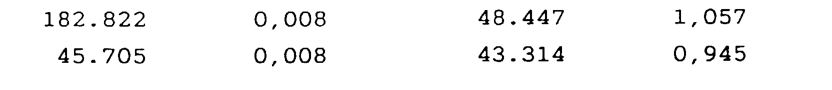

In diesem Reagenzsystem konnte eine Sensitivitätssteigerung mittels der luminometrischen Auswertung gegenüber der photometrischen Auswertung um einem Faktor 10 erzielt werden.

### Beispiel 8: Anwendung des Systems zur Bestimmung von HIV-1 p24-Antigen

### Materialien und Verfahren (Geräte wie in Beispiel 2)

### Reagenzien:

Der Retro-Tek HIV-1 p24 Antigen Elisa war von Cellular Products, Buffalo; Avidin β-Galactosidase war von Pierce, Resorufin-β-D-galactopyranosid von Lambda Probes, MgCl₂ x 6H₂O und NaCl von Merck, HEPES/BSA-Puffer von Codon.

### Versuch:

Der HIV-1 p24 Elisa wurde laut beigepackter Arbeitsvorschrift durchgeführt. Statt der Streptavidin-Peroxidase-Arbeits-lösung wurde Avidin β-Galactosidase in HEPES/BSA (20 mM HEPES, 0,5 M NaCl, pH 7,5, mit 1,0 mg/ml BSA) 1:10.000 verdünnt eingesetzt. Als Substratpuffer wurden für die luminometrische Bestimmung 100 µl (1 x 10⁻⁴ M Resorufin-β-D-galactopyranosid, 0,1 M NaCl, 2 mM MgCl₂, 5 mM Imidazol) verwendet und 90 min bei 37°C inkubiert. Danach wurden 100 µl in Röhrchen transferiert, 10 µl H₂O₂ (1 M Uvasol H₂O) zugesetzt, in das Luminometer transferiert, 100 µl einer Lösung von TCPO in Acetonitril zugesetzt (1 mM), manuell gestartet und im 10 s-Integralmodus aufgezeichnet. Als Waschpuffer wurde auch für die luminometrische Auswertung der im Kit beigepackte Puffer verwendet.

### Vergleich:

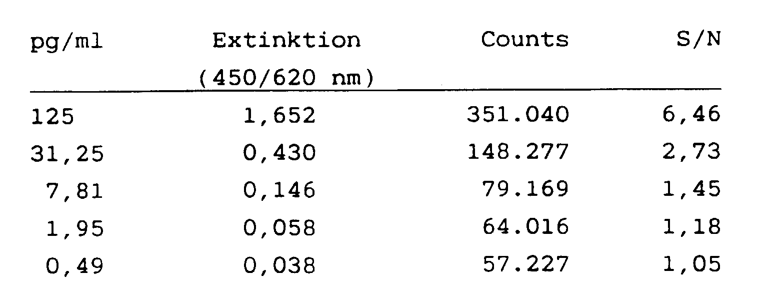

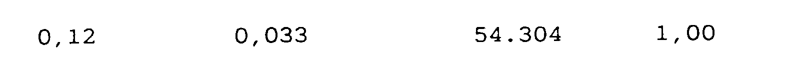

Aus diesem Vergleich ist ersichtlich, daß die luminometrische Auswertung unter sicherlich suboptimalen Bedingungen das gleiche Ergebnis bringt, wie die photometrische Auswertung. Dieses Beispiel zeigt, daß das System für Elisa-Techniken anwendbar ist.

## Patentansprüche

1. Verfahren zum Nachweis von Substanzen mittels auf Chemilumineszenz beruhenden Nachweisreaktionen in vorzugsweise protischen Medien, gegebenenfalls in Anwesenheit basischer Katalysatoren, wobei durch die Nachweisreaktion ein Luminophor durch Elektronentransfer von seinem nicht-angeregten in einen angeregten Zustand angehoben wird und anschließend die durch Zurückfallen des Luminophors in seinen nicht-angeregten Zustand emittierte Strahlung gemessen wird, dadurch gekennzeichnet, daß bei einer auf der oxidativen Zersetzung von gegebenenfalls substituierten Oxalaten oder Oxamiden beruhenden Chemilumineszenzreaktion ein redoxsensitives Luminophor verwendet wird, dessen beim Zurückfallen in den nichtangeregten Zustand emittierte und zu messende Strahlung langwelliger als die unspezifische Hintergrundstrahlung der verwendeten Reaktion ist und wobei die unspezifische Hintergrundstrahlung der Reaktion durch eine geeignete Wellenlängenselektionseinheit im wesentlichen zur Gänze gelöscht wird.

2. Verfahren nach Anspruch 1, dadurch gekennzeichnet, daß zusätzlich der Luminophor durch eine weitere chemische bzw. enzymatische Reaktion vor Durchführung der Chemilumineszenzreaktion gezielt akkumuliert wird.

3. Verfahren nach Anspruch 1 oder 2, dadurch gekennzeichnet, daß zunächst in wässeriger Lösung eine biochemische Umsetzung stattfindet und anschließend die Chemilumineszenzreaktion durch Zugabe der hierzu nötigen Reagentien in einem geeigneten, an sich bekannten aprotischen Lösungsmittel oder Lösungsmittelsystem, beispielsweise Acetonitril, erfolgt.

4. Verfahren nach einem der Ansprüche 1 bis 3, dadurch gekennzeichnet, daß bei der Peroxyoxalat-Chemilumineszenzreaktion ein redoxsensitives Luminophor verwendet wird, dessen beim Zurückfallen in den nicht-angeregten Zustand emittierte und zu messende Strahlung langwelliger als etwa 500 nm ist und daß eine Wellenlängenselektionseinheit verwendet wird, welche die Hintergrundstrahlung zwischen etwa 350 und 540 nm im wesentlichen zur Gänze löscht.

5. Verfahren nach Anspruch 4, dadurch gekennzeichnet, daß als Luminophor Resorufinat verwendet wird.

6. Verfahren nach Anspruch 4, dadurch gekennzeichnet, daß als Luminophor Tetraphenylporphyrin verwendet wird.

7. Verfahren nach Anspruch 4, dadurch gekennzeichnet, daß als Luminophor Rhodamin 123 verwendet wird.

8. Verfahren nach Anspruch 4, dadurch gekennzeichnet, daß als Luminophor Chlorophyll verwendet wird.

9. Verfahren nach Anspruch 4, dadurch gekennzeichnet, daß als Luminophor (Z,Z)-16,17-Difluorboryl-13-ethyl-2,3,7,8,12,14-hexamethyl-1,17-dihydro-15H-tripyrrin-1-on verwendet wird.

10. Verfahren nach einem der Ansprüche 1 bis 9, dadurch gekennzeichnet, daß als Wellenlängenselektionseinheit ein Kantenfilter verwendet wird.

11. Verfahren nach einem der Ansprüche 1 bis 9, dadurch gekennzeichnet, daß als Wellenlängenselektionseinheit ein Interferenzfilter verwendet wird.

12. Verfahren nach einem der Ansprüche 1 bis 9, dadurch gekennzeichnet, daß als Wellenlängenselektionseinheit ein Gitter verwendet wird.

13. Verfahren nach einem der Ansprüche 1 bis 9, dadurch gekennzeichnet, daß als Wellenlängenselektionseinheit ein Prisma verwendet wird.

14. Verfahren nach einem der Ansprüche 4 bis 13, dadurch gekennzeichnet, daß ein redoxsensitives Luminophor verwendet wird, dessen beim Zurückfallen in den nicht-angeregten Zustand emittierte und zu messende Strahlung langwelliger als etwa 500 nm ist, welches Luminophor aus einem geeigneten Substrat unter direktem Einfluß einer nachzuweisenden Menge einer Substanz freigesetzt wird.

15. Verfahren nach Anspruch 14, dadurch gekennzeichnet, daß ein aus einem Substrat und einem Luminophor bestehendes Fluorogen eingesetzt wird, wobei der Luminophor unter direktem Einfluß einer nachzuweisenden Menge eines Enzyms aktiviert bzw. vom Substrat abgespalten wird.

16. Verfahren nach Anspruch 15, dadurch gekennzeichnet, daß das Enzym direkt an einen Antikörper gebunden ist.

17. Verfahren nach Anspruch 15, dadurch gekennzeichnet, daß das Enzym indirekt an einen Antikörper gebunden ist.

18. Verfahren nach Anspruch 15, dadurch gekennzeichnet, daß das Enzym direkt an ein Antigen gebunden ist.

19. Verfahren nach Anspruch 15, dadurch gekennzeichnet, daß das Enzym indirekt an ein Antigen gebunden ist.

20. Verfahren nach Anspruch 15, dadurch gekennzeichnet, daß das Enzym direkt an eine Nukleinsäuresonde gebunden ist.

21. Verfahren nach Anspruch 15, dadurch gekennzeichnet, daß das Enzym indirekt an eine Nukleinsäuresonde gebunden ist.

22. Verfahren nach einem der Ansprüche 15 bis 21, dadurch gekennzeichnet, daß der verwendete Luminophor mittels alkalischer Phosphatase freigesetzt wird.

23. Verfahren nach einem der Ansprüche 15 bis 21, dadurch gekennzeichnet, daß der verwendete Luminophor mittels Meerrettich-peroxidase aktiviert wird.

24. Verfahren nach einem der Ansprüche 15 bis 21, dadurch gekennzeichnet, daß der verwendete Luminophor mittels β-Galactosidase freigesetzt wird.

25. Verfahren nach einem der Ansprüche 1 bis 14, dadurch gekennzeichnet, daß der verwendete Luminophor aus einem geeigneten Substrat unter indirektem Einfluß einer nachzuweisenden Menge einer Substanz aktiviert bzw. freigesetzt wird.

26. Verfahren nach Anspruch 25, dadurch gekennzeichnet, daß der indirekte Einfluß einer nachzuweisenden Menge eines Antigens bzw. Antikörpers auf den mit einem Enzym zur Freisetzung des Luminophors gekoppelten Antikörper bzw. Antigen über ein oder mehrere weitere Antigen-Antikörper-Reaktionen erfolgt.

27. Verfahren nach Anspruch 25 oder 26, dadurch gekennzeichnet, daß der indirekte Einfluß einer nachzuweisenden Menge eines Antigens bzw. Antikörpers auf den mit einem Enzym zur Freisetzung des Luminophors gekoppelten Antikörper bzw. Antigen über ein oder mehrere zwischengeschaltete Substanzen erfolgt, welche mit ein oder mehreren spezifischen Erkennungsstellen für mindestens eine weitere Verbindung versehen sind, welche weitere Verbindung(en) dann mit dem jeweiligen Enzym gekoppelt sind.

## Claims

1. A method of detecting substances by means of detection reactions based on chemiluminescence in, preferably, protic media, optionally in the presence of basic catalysts, wherein a luminophore is raised from its non-excited state into an excited state by electron transfer due to the detection reaction and subsequently the radiation emitted by the luminophore when falling back into its non-excited state is measured, characterized in that a redox sensitive luminophore is used in a chemiluminescence reaction based on the oxidative decomposition of optionally substituted oxalates or oxamides, whose radiation emitted while falling back into the non-excited state and to be measured is of longer wavelength than the nonspecific background radiation of the reaction used, and wherein the nonspecific background radiation of the reaction is quenched substantially completely by a suitable wavelength selection unit.

2. A method according to claim 1, characterized in that the luminophore additionally is purposefully accumulated by a further chemical or enzymatic reaction prior to carrying out the chemiluminescence reaction.

3. A method according to claim 1 or 2, characterized in that, at first, a biochemical reaction in aqueous solution occurs and subsequently the chemiluminescence reaction is effected by adding the necessary reagents in a suitable aprotic solvent or solvent system known per se, for instance, acetonitrile.

4. A method according to any one of claims 1 to 3, characterized in that a redox sensitive luminophore is used in the peroxyoxalate chemiluminescence reaction, whose radiation emitted when falling back into the non-excited state and to be measured is of a wavelength longer than approximately 500 nm, and that a wave length selection unit is used that quenches the background radiation between about 350 and 540 nm substantially completely.

5. A method according to claim 4, characterized in that resorufinate is used as said luminophore.

6. A method according to claim 4, characterized in that tetraphenyl porphyrin is used as said luminophore.

7. A method according to claim 4, characterized in that rhodamine 123 is used as said luminophore.

8. A method according to claim 4, characterized in that chlorophyll is used as said luminophore.

9. A method according to claim 4, characterized in that (Z,Z)-16,17-difluoroboryl-13-ethyl-2,3,7,8,12,14-hexamethyl-1,17-dihydro-15H-tripyrrin-l-one is used as said luminophore.

10. A method according to any one of claims 1 to 9, characterized in that a cut-off filter is used as said wavelength selection unit.

11. A method according to any one of claims 1 to 9, characterized in that an interference filter is used as said wavelength selection unit.

12. A method according to any one of claims 1 to 9, characterized in that a grid is used as said wavelength selection unit.

13. A method according to any one of claims 1 to 9, characterized in that a prism is used as said wavelength selection unit.

14. A method according to any one of claims 4 to 13, characterized in that a redox sensitive luminophore is used, whose radiation emitted when falling back into the non-excited state and to be measured is of longer wavelength than about 500 nm, which luminophore is released from a suitable substrate under the direct influence of a substance amount to be detected.

15. A method according to claim 14, characterized in that a fluorogen comprised of a substrate and a luminophore is used, the luminophore being activated or cleaved, respectively, from the substrate under the direct influence of an amount of enzyme to be detected.

16. A method according to claim 15, characterized in that the enzyme is directly bound to an antibody.

17. A method according to claim 15, characterized in that the enzyme is indirectly bound to an antibody.

18. A method according to claim 15, characterized in that the enzyme is directly bound to an antigen.

19. A method according to claim 15, characterized in that the enzyme is indirectly bound to an antigen.

20. A method according to claim 15, characterized in that the enzyme is directly bound to a nucleic acid probe.

21. A method according to claim 15, characterized in that the enzyme is indirectly bound to a nucleic acid probe.

22. A method according to any one of claims 15 to 21, characterized in that the luminophore used is set free by means of alkaline phosphatase.

23. A method according to any one of claims 15 to 21, characterized in that the luminophore used is activated by means of horseraddish peroxidase.

24. A method according to any one of claims 15 to 21, characterized in that the luminophore used is set free by means of β-galactosidase.

25. A method according to any one of claims 1 to 14, characterized in that the luminophore used is activated or set free, respectively, from a suitable substrate under the indirect influence of a substance amount to be detected.

26. A method according to claim 25, characterized in that the indirect influence of an amount of antigen or antibody to be detected on the antibody or antigen coupled with an enzyme for setting free the luminophore is effected via one or several additional antigen-antibody reactions.

27. A method according to claim 25 or 26, characterized in that the indirect influence of an amount of antigen or antibody to be deteced, on the antibody or antigen coupled with an enzyme for setting free the luminophore is effected via one or several intermediate substances provided with one or several specific identification sites for at least one further compound, which further compound(s) is (are) then coupled to the respective enzyme.

## Revendications

1. Procédé de mise en évidence de substances au moyen de réactions de mise en évidence reposant sur la chimioluminescence dans des milieux de préférence protiques, le cas échéant en présence de catalyseurs basiques, étant entendu que la réaction de mise en évidence fait passer, par transfert d'électrons, un luminophore de son état non excité à un état excité et qu'on mesure ensuite le rayonnement émis par retour du luminophore à son état non excité, caractérisé en ce qu'on utilise, au cours d'une réaction de chimioluminescence reposant sur la décomposition par oxydation d'oxalates ou d'oxamides éventuellement substitués, un luminophore sensible à une oxydoréduction dont le rayonnement à mesurer et émis lors du retour à l'état non excité a une longueur d'onde plus grande que le rayonnement de fond non spécifique de la réaction utilisée et étant entendu que le rayonnement de fond non spécifique de la réaction est essentiellement totalement éteint par une unité de sélection de longueur d'onde appropriée.

2. Procédé selon la revendication 1, caractérisé en ce que de plus le luminophore est accumulé de façon ciblée par une autre réaction chimique ou enzymatique avant réalisation de la réaction de chimioluminescence.

3. Procédé selon la revendication 1 ou 2, caractérisé en ce qu'une transformation biochimique a d'abord lieu dans une solution aqueuse et qu'ensuite se produit la réaction de chimioluminescence par addition des corps réagissants nécessaires dans un solvant ou système de solvant aprotique connu en soi approprié.

4. Procédé selon l'une des revendications 1 à 3, caractérisé en ce qu'on utilise, pour la réaction de chimioluminescence de peroxyoxalate, un luminophore sensible à une oxydoréduction dont le rayonnement à mesurer et émis lors du retour à l'état non excité a une longueur d'onde supérieure à environ 500 nm et qu'on utilise une unité de sélection de longueur d'onde qui éteint essentiellement totalement le rayonnement de fond entre environ 350 et 540 nm.

5. Procédé selon la revendication 4, caractérisé en ce qu'on utilise le résorufinate en tant que luminophore.

6. Procédé selon la revendication 4, caractérisé en ce qu'on utilise la tétraphénylporphyrine en tant que luminophore.

7. Procédé selon la revendication 4, caractérisé en ce qu'on utilise la rhodamine 123 en tant que luminophore.

8. Procédé selon la revendication 4, caractérisé en ce qu'on utilise la chlorophylle en tant que luminophore.

9. Procédé selon la revendication 4, caractérisé en ce qu'on utilise la (Z,Z)-16,17-difluoroboryl-13-éthyl-2,3,7,8,12,14-hexaméthyl-1,17-dihydro-15H-tripyrrin-1-one en tant que luminophore.

10. Procédé selon l'une des revendications 1 à 9, caractérisé en ce qu'on utilise un filtre à arêtes en tant qu'unité de sélection de longueur d'onde.

11. Procédé selon l'une des revendications 1 à 9, caractérisé en ce qu'on utilise un filtre d'interférence en tant qu'unité de sélection de longueur d'onde.

12. Procédé selon l'une des revendications 1 à 9, caractérisé en ce qu'on utilise un grillage en tant qu'unité de sélection de longueur d'onde.

13. Procédé selon l'une des revendications 1 à 9, caractérisé en ce qu'on utilise un prisme en tant qu'unité de sélection de longueur d'onde.

14. Procédé selon l'une des revendications 4 à 13, caractérisé en ce qu'on utilise un luminophore sensible à une oxydoréduction dont le rayonnement à mesurer et émis lors du retour à l'état non excité a une longueur d'onde supérieure à environ 500 nm, lequel luminophore est libéré d'un substrat approprié sous l'action directe d'une quantité à mettre en évidence d'une substance.

15. Procédé selon la revendication 14, caractérisé en ce qu'on utilise un fluorogène constitué d'un substrat et d'un luminophore, étant entendu que le luminophore, sous l'action directe d'une quantité à mettre en évidence d'une enzyme, est activé ou séparé du substrat.

16. Procédé selon la revendication 15, caractérisé en ce l'enzyme est liée de façon directe à un anticorps.

17. Procédé selon la revendication 15, caractérisé en ce l'enzyme est liée de façon indirecte à un anticorps.

18. Procédé selon la revendication 15, caractérisé en ce l'enzyme est liée de façon directe à un antigène.

19. Procédé selon la revendication 15, caractérisé en ce l'enzyme est liée de façon indirecte à un antigène.

20. Procédé selon la revendication 15, caractérisé en ce l'enzyme est liée de façon directe à une sonde d'acides nucléiques.

21. Procédé selon la revendication 15, caractérisé en ce l'enzyme est liée de façon indirecte à une sonde d'acides nucléiques.

22. Procédé selon l'une des revendications 15 à 21, caractérisé en ce que le luminophore utilisé est libéré à l'aide de phosphatase alcaline.

23. Procédé selon l'une des revendications 15 à 21, caractérisé en ce que le luminophore utilisé est activé à l'aide de peroxydase de raifort.

24. Procédé selon l'une des revendications 15 à 21, caractérisé en ce que le luminophore utilisé est libéré à l'aide de β-galactosidase.

25. Procédé selon l'une des revendications 1 à 14, caractérisé en ce que le luminophore utilisé est activé ou libéré à partir d'un substrat approprié sous l'action indirecte d'une quantité à mettre en évidence d'une substance.

26. Procédé selon la revendication 25, caractérisé en ce que l'action indirecte d'une quantité à mettre en évidence d'un antigène ou anticorps sur l'anticorps ou antigène couplé avec une enzyme en vue de la libération du luminophore se produit par l'intermédiaire d'une ou plusieurs autres réactions antigène-anticorps.

27. Procédé selon la revendication 25 ou 26, caractérisé en ce que l'action indirecte d'une quantité à mettre en évidence d'un antigène ou anticorps sur l'anticorps ou antigène couplé avec une enzyme en vue de la libération du luminophore se produit par l'intermédiaire d'une ou plusieurs substances intercalées qui sont dotées d'un ou plusieurs sites de reconnaissance spécifiques pour au moins un autre composé, lequel ou lesquels un ou plusieurs autres composés est ou sont ensuite couplé(s) avec l'enzyme correspondante.
